# EUROPEAN PATENT SPECIFICATION

(11) **EP 1 694 299 B1**
(45) Date of publication and mention of the grant of the patent: **20.02.2008**
(21) Application number: 04799349.8
(22) Date of filing: 14.11.2004
(51) Int. Cl.: A61K 9/127

(54) **METHOD FOR DRUG LOADING IN LIPOSOMES**
VERFAHREN FÜR DIE ARZNEIBELADUNG IN LIPOSOMEN
PROCEDE POUR CHARGER UN MEDICAMENT EN LIPOSOMES

(30) Priority: 14.11.2003 US 520205 P
(43) Date of publication of application: 30.08.2006
(73) Proprietor: YISSUM RESEARCH DEVELOPMENT COMPANY OF THE HEBREW UNIVERSITY OF JERUSALEM, 91390 Jerusalem (IL)
(72) Inventor: BARENHOLZ, Yechezkel, 93707 Jerusalem (IL); GABIZON, Alberto A., 96920 Jerusalem (IL)
(74) Representative: Williams, Paul Edwin
(86) International application number: PCT/IL2004/001041
(87) International publication number: WO 2005/046643

(56) References cited:
- US-A- 4 954 345
- US-A- 5 013 556
- US-A- 5 169 636
- US-A- 5 192 549
- DATABASE WPI Section Ch, Week 199423 Derwent Publications Ltd., London, GB; Class B05, AN 1994-186364 XP002324962 & JP 06 122634 A (NIPPON SEIKA KK) 6 May 1994 (1994-05-06)

## Description

### Field of the Invention

The present invention relates to a method and the product obtained thereby of loading therapeutic agents into preformed liposomes, in particular, loading of protonatable compounds by an ammonium ion gradient having glucuronate as the balancing anion.

### Background of the Invention

Delivery of therapeutic agents via liposomal compositions has drastically changed the drug pharmacokinetics and biodistribution of some agents (Martin, F.M., in MEDICAL APPLICATIONS OF LIPOSOMES, Lasic, D. D. and D. Papahadjopaulos, eds., p. 635-88, Elsevier, Amsterdam (1998)). For example, doxorubicin, which is known for its dose limiting cardiac-toxicity, shows no apparent (clinical and functional) cardiac-toxicity in patients with solid tumors when administered entrapped in liposomes (Doxil^{®}, ALZA Corporation, Mountain View, CA; Uziely, B. et al., J. Clin. Onco., 13:1777-1785 (1995); Working, P.K. et al., J. Pharmaco. Exp. Ther., 289:1128-1133 (1999)). A cardiac biopsy study of acquired immune deficiency syndrome (AIDS)-related Kaposi sarcoma (KS) patients receiving large cumulative dosages of Doxil*®* showed no tissue damage, which suggests that the liposomal formulation may have a cardioprotective effect on doxorubicin (Berry, G. et al., Ann. Oncol., 9:71-76 (1998)). The lack of cardiac-toxicity is attributed, in part, to the long circulation half-life of liposomes (polyethylene glycol coated liposomes known as Stealth*®*, ALZA Corporation, Mountain View, CA) and the stable drug retention, such that most of the administered dose reaches tissues in liposome-encapsulated form with only minimal amounts of drug (< 5%) leaking from liposomes during circulation and distributed to tissue as free drug (Martin, F.M., *supra,* (1998); Gabizon, A. et al., Cancer Res., 54:987-92 (1994)).

It is known that long-circulating liposomes accumulate preferentially (10 fold) in tissues with increased microvascular permeability, which includes most tumors with active neoangiogenesis (Wu, N.Z., et al., Cancer Res., 53:3765-3770 (1993); Yuan, F., et al., Cancer Res., 54:3352-3356 (1994)). Long circulating liposomes also accumulate in various healthy and susceptible tissues such as the skin (Gabizon, A. et al., Adv. Drug Deliv. Rev., 24:337-344 (1997)) and probably the mucosas. On prolonged exposure, accumulation of liposome-entrapped doxorubicin in the skin may cause palmer-plantar erythrodysestheris (PPE, also known as hand-foot syndrome; Lyass et al., Cancer 89:1037-1047 (2000)). The onset of PPE may be prevented by prolongation of dosing intervals, however, dose and/or schedule modifications may reduce efficacy against certain tumors, e.g., breast carcinoma (Lyass *et al., supra,* (2000); Ranson, M.R. et al., J. Clin. Oncol., 15:3185-3191 (1997)).

Current preclinical and clinical data on the long circulating, liposome-entrapped doxorubicin (Doxil^{®}) indicate that there is negligible release of drug from circulating liposomes (<5% of the injected dose). Once the liposomes have extravasated into extracellular tissue fluids, little is known of the processes determining drug release. It is believed that gradual loss of the proton gradient retaining the drug, enzymatic breakdown of liposomal phospholipids by phospholipases, and/or endocytosis by scavenger macrophages likely contribute to drug release. Doxorubicin when entrapped in the commercially -available liposomal Doxil^{®} forms a salt with the divalent sulfate anion. The salt precipitates or gels due to its low solubility in the aqueous internal liposomal compartment. This gel formation stabilizes the entrapped doxorubicin in the lipid vesicle and decreases its rate of efflux.

Altering the holding capability of the anion on doxorubicin could have a major impact on the rate of drug release. For example, accelerating the rate of drug release from Doxil^{®} liposomes, without interfering with its long-circulating, tumor-homing properties, may be of significance for the following reasons: (1) the tumor-inhibitory activity may increase because of more time-intense exposure of tumors to the drug, and (2) the skin toxicity may decrease because this class of toxicity is mainly a function of prolonged exposure of skin tissues to the drug.

Accordingly, a liposome composition that varied the release of an entrapped compound, and in particular, doxorubicin, from liposomes is desirable. A method for entrapping therapeutic compounds in preformed liposomes which retains the advantages of the ammonium sulfate gradient, e.g., efficiency and stability, yet enables the entrapped compound to be release at a higher rate would be desirable.

### Summary of the Invention

In one aspect, the invention provides a liposomal composition liposomes comprised of vesicle forming lipids and having an entrapped ionizable therapeutic agent in association with a glucuronate anion. The therapeutic agent so loaded has a higher release rate than that loaded by an ammonium gradient having sulfate as the balancing, or counter, anion.

In one embodiment, the vesicle-forming lipids forming the liposomes are phospholipids. In another embodiment, the liposomes further comprise between about 1-20 mole percent of a vesicle-forming lipid derivatized with a hydrophilic polymer, such as polyethylene glycol.

In another embodiment, the vesicle-forming lipid is hydrogenated soy phosphatidylcholine (HSPC) and said vesicle-forming lipid derivatized with a hydrophilic polymer is distearoyl phosphatidylethanolamine (DSPE) derivatized with polyethylene glycol. In yet another embodiment, the liposomes further comprise cholesterol. An exemplary composition is HSPC, cholesterol, and DSPE-PEG in a molar ratio of is 92.5:70:7.5.

In another embodiment, the therapeutic agent is an anthracycline antibiotic. Exemplary anthracycline antibiotic include doxorubicin, daunorubicin, and epirubicin.

The composition described above is used, in another aspect, for treating a patient. The composition is used, in another aspect, for treating a neoplasm in a patient.

In another aspect, the invention includes an improved method of preparing liposomes that have an entrapped ionizable therapeutic agent, where the therapeutic agent is loaded into pre-formed liposomes against an ammonium ion gradient with sulfate as a counterion. The improvement comprises loading the ionizable therapeutic agent into liposomes by an ammonium ion gradient having glucuronate as a counterion.

In this improved method, loading includes preparing a suspension of liposomes, each liposome having at least one internal aqueous compartment that contains ammonium glucuronate at a first concentration, in one embodiment.

In another embodiment, the improved method includes preparing liposomes suspended in an external bulk medium having a second concentration of ammonium glucuronate, wherein the first concentration is higher than the second concentration thereby establishing an ammonium ion concentration gradient across lipid bilayers of the liposomes.

In another embodiment, the improved method includes adding an amount of the therapeutic agent to the suspension of liposomes.

In another aspect, the invention includes a method of preparing liposomes, comprising forming liposomes having an internal compartment and a bilayer lipid membrane. The liposomes have a concentration gradient of ammonium glucuronate across their bilayer lipid membranes. The, the liposomes are contacted with an ionizable therapeutic agent to achieve transport of the agent into the internal compartment.

In one embodiment, the method includes (i) preparing a suspension of liposomes, each liposome in the suspension having at least one internal aqueous compartment that contains ammonium glucuronate at a first concentration, the liposomes suspended in an external bulk medium comprising ammonium glucuronate at the first concentration; (ii) reducing the first concentration of ammonium glucuronate in the external bulk medium to a lower, second concentration of ammonium glucuronate, thereby establishing an ammonium ion concentration gradient across lipid bilayers of the liposomes.

In various embodiments, the step of reducing is achieved by dilution, dialysis, diafiltration, or ion exchange.

In still another aspect, the invention includes a method for loading a protonatable compound into pre-formed liposomes, comprising preparing a suspension of liposomes having a greater concentration of ammonium glucuronate inside the liposomes than outside the liposomes thereby establishing an ammonium ion concentration gradient from the inside to outside of the liposomes. The gradient is capable of active transport of said protonatable compound towards the inside of the liposomes. The method also includes adding an amount of protonatable compound to the suspension, and allowing the protonatable compound to transport into the liposomes to achieve a content of said protonatable compound inside the liposomes to be greater than that outside of the liposomes.

In one embodiment, the method includes forming the liposomes in the presence of an ammonium glucuronate solution having a first concentration; and entrapping said ammonium glucuronate solution of said first concentration inside said liposomes; and reducing said first concentration of said ammonium glucuronate solution outside of the liposomes to a second concentration which is less than that of said first concentration.

The method of the invention has a high loading efficiency. In one embodiment greater than 50% of the amount of protonatable compound added to the suspension is transported to the inside of the liposomes. In another embodiment approximately 90% of the amount of protonatable compound added to the suspension is transported to the inside of the liposomes. In specific embodiments, the loading efficiency for doxorubicin is greater than 90% and the doxorubicin to phospholipid ratio is in the range of about 100-150 µg/µmol.

These and other objects and features of the invention will be more fully appreciated when the following detailed description of the invention is read in conjunction with the accompanying drawings.

### Brief Description of the Drawings

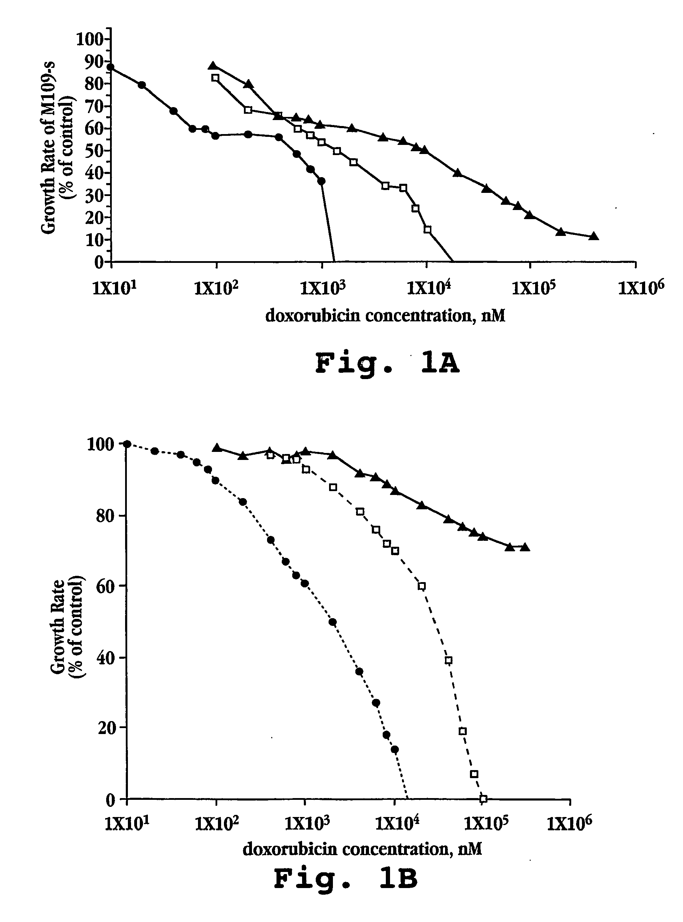

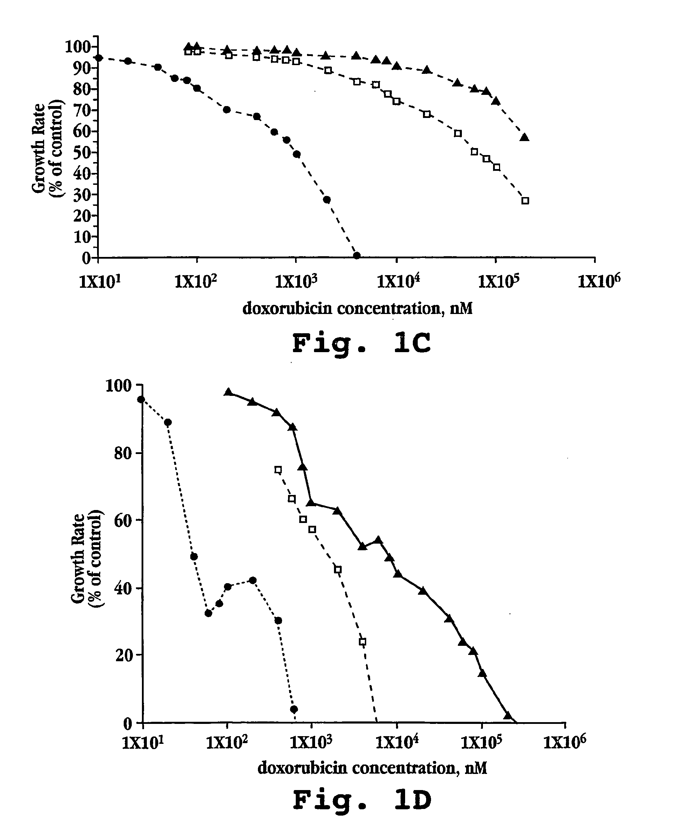

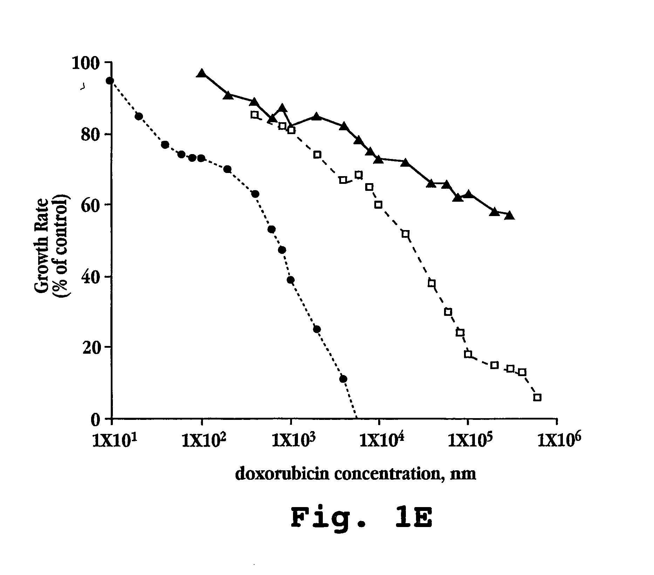

Figs. 1A-1E are growth inhibition curves plotting the growth rate, as a percent of untreated control cells, of mouse cell lines M109ST (Fig. 1A), M109R (Fig. 1B) and of human cell lines C-26 (Fig. 1C), KB (Fig. 1D), and KB-V (Fig. 1E), against doxorubicin concentration (in nM), after treatment with free doxorubicin (circles), liposome-entrapped doxorubicin, where the doxorubicin was remotely loaded into the liposomes against an ammonium sulfate gradient (triangles, "lipo-dox-AS") or against an ammonium glucuronate gradient (squares, "lipo-dox-AG");

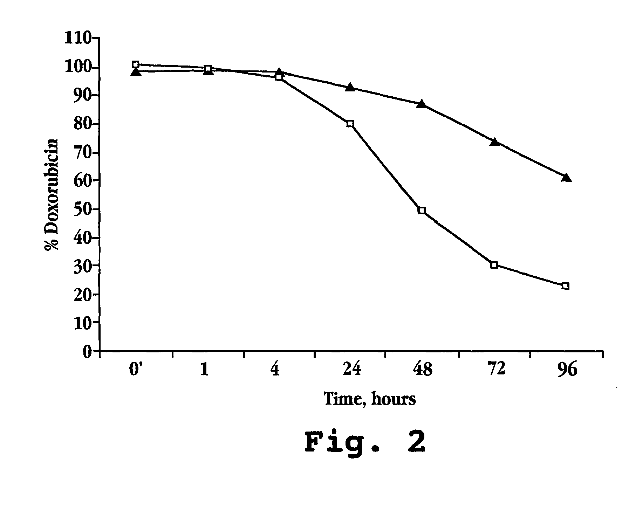

Fig. 2 shows the *in vitro* leakage rate of doxorubicin from liposomes, where the doxorubicin was remotely loaded into the liposomes against an ammonium sulfate gradient (triangles, "lipo-dox-AS") or against an ammonium glucuronate gradient (squares, "lipo-dox-AG");

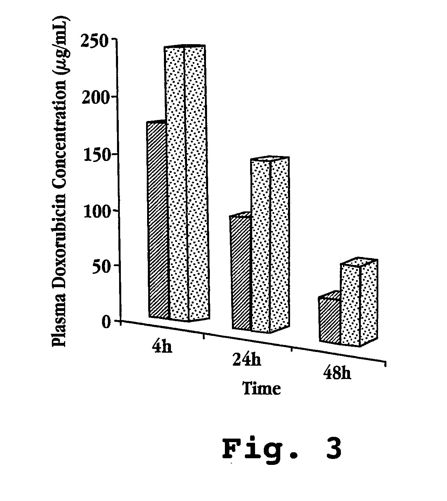

Fig. 3 is a bar graph showing doxorubicin concentration (µg/mL) in mouse plasma at various times after the injection of liposomes containing doxorubicin, where the doxorubicin was remotely loaded into the liposomes against an ammonium sulfate gradient dotted bars) or against an ammonium glucuronate gradient (hatched bars);

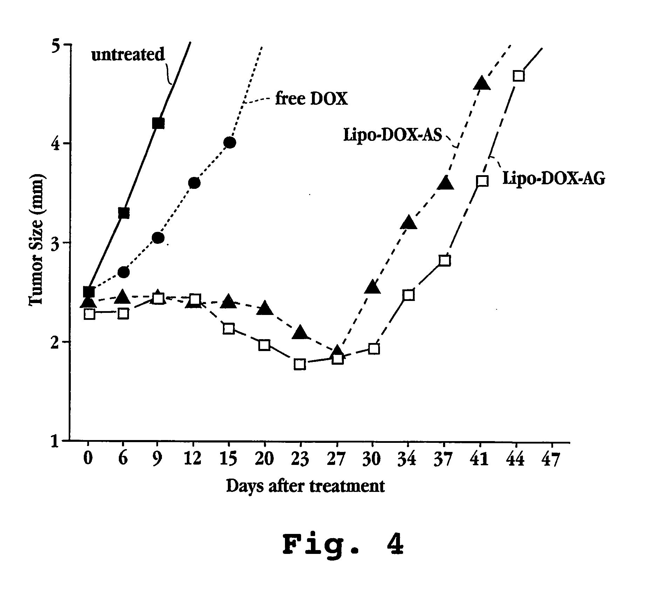

Fig. 4 is a plot of mean footpad thickness, in mm, in mice inoculated with M109-S cells as a function of days after treatment with saline (closed squares), free doxorubicin (circles), or doxorubicin entrapped in liposomes, where the doxorubicin was remotely loaded Into the liposomes against an ammonium sulfate gradient (triangles, "lipo-dox-AS") or against an ammonium glucuronate gradient (open squares, "lipo-dox-AG");

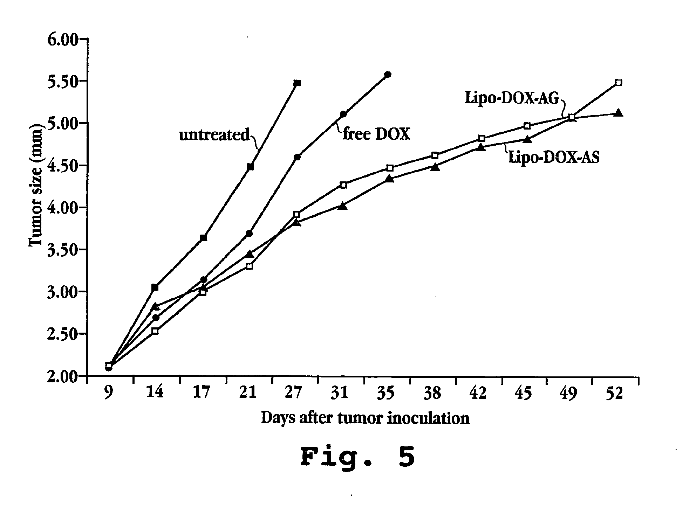

Fig. 5 is a plot of mean footpad thickness, in mm, in mice inoculated with M109R cells (doxorubicin-resistant tumor cells) as a function of days after treatment with saline (closed squares), free doxorubicin (circles), or doxorubicin entrapped in liposomes, where the doxorubicin was remotely loaded into the liposomes against an ammonium sulfate gradient (triangles, "lipo-dox-AS") or against an ammonium glucuronate gradient (open squares, "lipo-dox-AG"); and

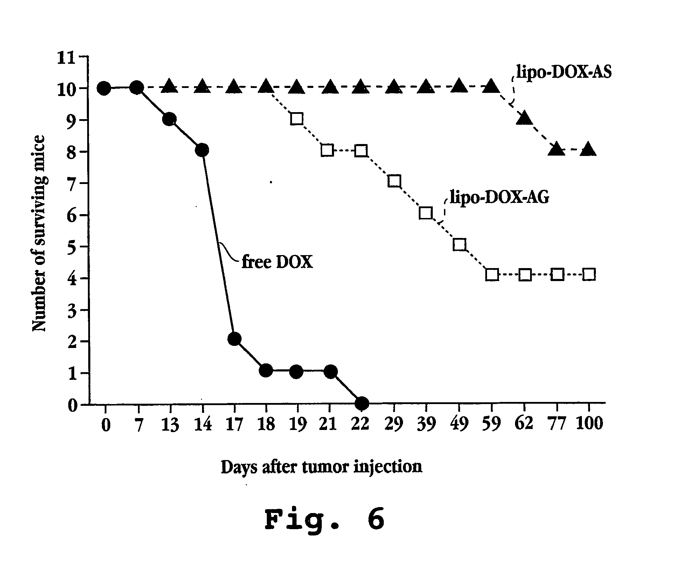

Fig. 6 is a plot of number of surviving mice as a function of days after inoculation with C-26 tumor cells and treatment with free doxorubicin (circles) or with doxorubicin entrapped in liposomes, where the doxorubicin was remotely loaded into the liposomes against an ammonium sulfate gradient (triangles, "lipo-dox-AS") or against an ammonium glucuronate gradient (squares, "lipo-dox-AG").

### Detailed Description of the Invention

The invention provides a liposomal compositon where an ionizable therapeutic agent is entrapped in the internal liposomal compartment(s) in the form of an ionic salt with monovalent glucuronate anions. As will be shown below, the entrapped therapeutic agent has a faster release rate from the liposomes compared to the release rate of the agent entrapped in the liposomes in the form of an ionic salt with divalent sulfate anions. The invention also provides a remote loading procedure for loading therapeutic agents into pre-formed liposomes against an ammonium glucuronate gradient. The faster rate of release of the therapeutic agent from the liposomes affords flexibility to adjust dosing schedules without compromising the biological efficacy of the therapeutic agents. The method of the invention therefore provides a beneficial alternative to loading by ammonium sulfate.

Similar to the conventional ammonium sulfate gradient method, the ammonium glucuronate remote loading method does not require the liposomes to be prepared in acidic pH, nor to alkalinize the extraliposomal aqueous medium. The approach also permits the loading of therapeutic agents in a broad spectrum of liposomes of various types, sizes, and compositions, including sterically-stabilized liposomes, immunoliposomes, and sterically-stabilized immunoliposomes. "Entrapped" as used herein refers to an agent entrapped within the aqueous spaces of the liposomes or within the lipid bilayers.

The higher release rate is a result of using glucuronate as the balancing anion. While not wishing to be bound by theory, it is hypothesized that the glucuronate ion, being monovalent and containing several hydroxyl functional groups on its six-membered ring, is less effective compared to a sulfate ion at inducing aggregation and precipitation of the therapeutic agent after being transported inside the liposomes. The inventors have observed that the solubility of doxorubicin is approximately 100-fold greater in a 250 mM ammonium glucuronate (AG) solution than in a 250 mM ammonium sulfate (AS) solution. In addition, doxorubicin precipitates at less than 2 mM concentration in the presence of sulfate ions, while a much higher concentration of doxorubicin is required for precipitation to occur in the presence of glucuronate ions. Accordingly, when glucuronate is the balancing anion, more of the therapeutic agent is in a soluble form and therefore it is more available for release from the liposomes. Further, the permeability of glucuronate through the liposomal membranes is very low, possibly due to its low pKa, its bulkiness and/or polarity, making it very efficient for maintaining the ammonium ion gradient for loading of the therapeutic agents.

The method of the invention can be used to remotely load essentially any therapeutic agent which is protonatable (can exist in a positively charged state) when dissolved in an appropriate aqueous medium. Preferably, the agent should be relatively lipophilic so that it will partition into the lipid vesicle membranes. Also, preferably, the therapeutic compound for loading is a weak amphipathic compound, that is a compound having either weak basic or acidic moieties. Examples of therapeutic agents which can be loaded into liposomes by the method of the invention include, but are not limited to, doxorubicin, mitomycin, bleomycin, daunorubicin, streptozocin, vinblastine, vincristine, mechlorethamine hydrochloride, melphalan, cyclophosphamide, triethylenethiophosphoramide, carmustine, lomustine, semustine, fluoruracil, hydroxyurea, thioguanine, cytarabine, floxuridine, decarbazine, cisplatin, procarbazine, ciprofloxacin, epirubicin, carcinomycin, N-acetyladriamycin, rubidazone, 5-imidodaunomycin, N-acetyldaunomycine, all anthracyline drugs, daunoryline, propranolol, pentamindine, dibucaine, tetracaine, procaine, chlorpromazine, pilocarpine, physostigmine, neostigmine, chloroquine, amodiaquine, chloroguanide, primaquine, mefloquine, quinine, pridinol, prodipine, benztropine mesylate, trihexyphenidyl hydrochloride, propranolol, timolol, pindolol, quinacrine, benadryl, promethazine, dopamine, serotonin, epinephrine, codeine, meperidine, methadone, morphine, atropine, decyclomine, methixene, propantheline, imipramine, amitriptyline, doxepin, desipramine, quinidine, propranolol, lidocaine, chlorpromazine, promethazine, perphenazine, acridine orange, prostaglandins, fluorescein, carboxyfluorescein, and other molecules similar to these above.

In addition to loading a single therapeutic agent, the method can be used to load multiple therapeutic agents, either simultaneously or sequentially. Also, the liposomes into which the protonatable therapeutic agents are loaded can themselves be pre-loaded with other pharmaceutical agents or drugs using conventional encapsulation techniques (e.g., by incorporating the drug in the buffer from which the liposomes are prepared). The method of the invention therefore provides great flexibility in preparing liposome encapsulated "drug cocktails" for use in therapies. Of course, if desired, one or more of the protonatable drugs listed above can be pre-loaded and then the same or a different drug can be added to the liposomes using the ammonium glucuronate gradient of the present invention.

The method is particularly suitable for loading weakly amphipathic drugs such as doxorubicin. Doxorubicin loaded in liposomes having an external surface coating of hydrophilic polymer chains by an ammonium glucuronate gradient (referred to herein as "lipo-dox-AG") exhibits a faster release rate than doxorubicin loaded in liposomes having an external surface coating of hydrophilic polymer chains by an ammonium sulfate gradient (referred to herein as "lipo-dox-AS"; commercially known as Doxil^{®}), and has similar biological efficacy. It is contemplated that the faster release of drug when loaded into liposomes against an ammonium glucuronate gradient lessens the duration of the drug in the blood and lowers the opportunity for doxorubicin to accumulate in the skin to cause palmar-plantar erythrodysesthesia (PPE, also known as hand-foot syndrome), a side effect observed with liposomal-entrapped doxorubicin is administered.

In studies performed in support of the invention, liposomes containing entrapped doxorubicin were prepared, where the doxorubicin was remotely loaded into preformed liposomes against an ammonium sulfate gradient or against an ammonium glucuronate gradient. In Section I below, the liposome composition and the remote loading procedure will be described. These liposomes were characterize *in vitro* to determine their cytotoxicity, cellular drug uptake, and plasma leakage rate, also described in Section I. In Sections II and III, the *in vivo* plasma clearance rate and the therapeutic activity of the liposome-entrapped doxorubicin are discussed.

### I. Liposome Components and Preparation

### A Liposome Component

Liposomes suitable for use in the compositions of the present invention include those composed primarily of vesicle-forming lipids. Vesicle-forming lipids, exemplified by the phospholipids, form spontaneously into bilayer vesicles in water at physiological pH and temperatures. The liposomes can also include other lipids, incorporated into the lipid bilayers, with the hydrophobic moiety in contact with the interior, hydrophobic region of the bilayer membrane, and the head group moiety oriented toward the exterior, polar surface of the bilayer membrane.

The vesicle-forming lipids are preferably ones having two hydrocarbon chains, typically acyl chains, and a head group, either polar or nonpolar. There are a variety of diacyl synthetic vesicle-forming lipids and naturally-occurring vesicle-forming lipids, such as phospholipids, diglycerides, dialiphatic glycolipids, single lipids such as sphingomyelin and glycosphingolipid, cholesterol and derivatives thereof, alone or in combinations and/or with or without liposome membrane rigidifying agents. As defined herein, "phospholipids" include phosphatidylcholine (PC), phosphatidylethanolamine (PE), phosphatidic acid (PA), phosphatidylinositol (PI), phosphatidylserine (PS), sphingomyelin, plasmalogens, and phosphatidylcholine lipid derivatives where the two hydrocarbon chains are typically between about 14-22 carbon atoms in length, and have varying degrees of unsaturation. The above-described lipids and phospholipids whose acyl chains have varying degrees of saturation can be obtained commercially or prepared according to published methods.

Cationic lipids are also suitable for use in the liposomes of the invention, where the cationic lipid can be included as a minor component of the lipid composition or as a major or sole component. Such cationic lipids typically have a lipophilic moiety, such as a sterol, an acyl or diacyl chain, and where the lipid has an overall net positive charge. Preferably, the head group of the lipid carries the positive charge. Exemplary cationic lipids include 1,2-dioleyloxy-3-(trimethylarnino) propane (DOTAP); N-[I-(2,3,-ditetradecyloxy)propyl]-NN-dimethyl-N-hydroxyethylanimonium bromide (DMRIE); N-[I-(2,3,-dioleyloxy)propyl]-NN-dimethyl-N-hydroxy ethylammonium bromide (DORIE); N-[1-(2,3-dioleyloxy) propyl]-N,N,N-trimethylammoniurn chloride (DOTMA); 30[N-(N',N'-dirnethylaminoethane) carbarnoly] cholesterol (DC-Chol); and dimethyldioctadecylammonium (DDAB).

The cationic vesicle-forming lipid may also be a neutral lipid, such as dioleoylphosphafidyl ethanolamine (DOPE) or an amphipathic lipid, such as a phospholipid, derivatized with a cationic lipid, such as polylysine or other polyamine lipids. For example, the neutral lipid (DOPE) can be derivatized with polylysine to form a cationic lipid.

The vesicle-forming lipid can be selected to achieve a specified degree of fluidity or rigidity, to control the stability of the liposome in serum and to control the rate of release of the entrapped agent in the liposome. Liposomes having a more rigid lipid bilayer, or a liquid crystalline bilayer, are achieved by incorporation of a relatively rigid lipid, e.g., a lipid having a relatively high phase transition temperature, e.g., above room temperature, more preferably above body temperature and up to 80°C. Rigid, *i*.*e*., saturated, lipids contribute to greater membrane rigidity in the lipid bilayer. Other lipid components, such as cholesterol, are also known to contribute to membrane rigidity in lipid bilayer structures.

Lipid fluidity is achieved by incorporation of a relatively fluid lipid, typically one having a lipid phase with a relatively low liquid to liquid-crystalline phase transition temperature, e.g., at or below room temperature, more preferably, at or below body temperature.

The liposomes may optionally include a vesicle-forming lipid derivatized with a hydrophilic polymer, as has been described, for example in U.S. Patent No. 5,013,556 and in WO 98/07409, which are hereby incorporated by reference. Incorporation of a hydrophilic polymer-lipid conjugate into the liposomal bilayer polymer provides a surface coating of hydrophilic polymer chains on both the inner and outer surfaces of the liposome lipid bilayer membranes. The outermost surface coating of hydrophilic polymer chains is effective to extend the blood circulation lifetime *in vivo* relative to liposomes lacking the polymer chain coating. The inner coating of hydrophilic polymer chains extends into the aqueous compartments in the liposomes, *i*.*e*., between the lipid bilayers and into the central core compartment, and is in contact with any entrapped agents. Vesicle-forming lipids suitable for derivatization with a hydrophilic polymer include any of those lipids listed above, and, in particular phospholipids, such as distearoyl phosphatidylethanolamine (DSPE).

Hydrophilic polymers suitable for derivatization with a vesicle-forming lipid include polyvinylpyrrolidone, polyvinylmethylether, polymethyloxazoline, polyethyloxazoline, polyhydroxypropyloxazoline, polyhydroxypropylmethacrylamide, polymethacrylainide, polydirnethylacrylamide, polyhydroxypropyhnethacrylate, polyhydroxyethylacrylate, hydroxymethylcellulose, hydroxyethylcellulose, polyethyleneglycol, and polyaspartamide. The polymers may be employed as homopolymers or as block or random copolymers.

A preferred hydrophilic polymer chain is polyethyleneglycol (PEG), preferably as a PEG chain having a molecular weight between about 500 and about 10,000 Daltons, more preferably between about 500 and about 5,000 Daltons, most preferably between about 1,000 to about 2,000 Daltons. Methoxy or ethoxy-capped analogues of PEG are also preferred hydrophilic polymers, commercially available in a variety of polymer sizes, e.g., 120-20,000 Daltons.

Preparation of vesicle-forming lipids derivatized with hydrophilic polymers has been described, for example in U.S. Patent No. 5,395,619. Preparation of liposomes including such derivatized lipids has also been described, where typically, between 1-20 mole percent of such a derivatized lipid is included in the liposome formulation. It will be appreciated that the hydrophilic polymer may be stably coupled to the lipid, or coupled through an unstable linkage which allows the coated liposomes to shed the coating of polymer chains as they circulate in the bloodstream or in response to a stimulus, as has been described, for example, in U.S. Patent No. 6,043,094, which is incorporated by reference herein.

### B. Liposome Preparation

Liposomal suspensions comprised of liposomes having an ion gradient across the liposome bilayer (also referred to as a 'transmembrane gradient') for use in remote loading can be prepared by a variety of techniques, such as those detailed in Szoka, F., Jr., et al., Ann Rev Biophys Bioeng 9:467, (1980). Multilarnellar vesicles (MLVs) can be formed by simple lipid-film hydration techniques. In this procedure, a mixture of liposome-forming lipids of the type described above is dissolved in a suitable organic solvent and the solvent is later evaporated off leaving behind a thin film. The film is then covered by an aqueous medium, containing the solute species, *e*.*g*., ammonium glucuronate, which forms the aqueous phase in the liposome interior spaces and also the extraliposomal suspending solution. The lipid film hydrates to form MLVs, typically with sizes between about 0.1 to 10 microns.

The lipids used in forming the liposomes of the present invention are preferably present in a molar ratio of about 70-100 mole percent vesicle-forming lipids, optionally 1-20 mole percent of a lipid derivatized with a hydrophilic polymer chain. One exemplary formulation includes 80-90 mole percent phosphatidylethanolamine, 1-20 mole percent of PEG-DSPE. Cholesterol may be included in the formulation at between about 1-50 mole percent. In a preferred embodiment, the lipid components are hydrogenated soy phosphatidylcholine (HSPC), cholesterol (Chol) and methoxy-capped polyethylene glycol derivatized distearyl phosphatidylethanolamine (mPEG(2000)-DSPE) in a molar ratio of 92.5:70:7.5.

For preparation liposomes having an ammonium glucuronate gradient, the hydration medium contains ammonium glucuronate. The concentration of ammonium glucuronate would depend on the amount of therapeutic agent to be loaded. Typically, the concentration is between 100 to 300 mM of ammonium glucuronate. In one preferred embodiment, the hydration medium contains 250 mM ammonium glucuronate.

The vesicles formed by the thin film method may be sized to achieve a size distribution within a selected range, according to known methods. Preferably, the liposomes are uniformly sized to a size range between 0.04 to 0.25 µm. Small unilamellar vesicles (SUVs), typically in the 0.04 to 0.08 µm range, can be prepared by post-formation sonication or homogenization. Homogeneously sized liposomes having sizes in a selected range between about 0.08 to 0.4 µm can be produced, e.g., by extrusion through polycarbonate membranes or other defined pore size membranes having selected uniform pore sizes ranging from 0.03 to 0.5 µm, typically, 0.05, 0.08, 0.1, or 0.2 µm. The pore size of the membrane corresponds roughly to the largest size of liposomes produced by extrusion through that membrane, particularly where the preparation is extruded two or more times through the same membrane. The sizing is preferably carried out in the original lipid-hydrating buffer, so that the liposome interior spaces retain this medium throughout the initial liposome processing steps. Preparation of an exemplary liposomal formulation is described in Example 1.

Generally, a therapeutic agent is loaded into the liposomes after sizing. A "remote" or "active" loading process results from exchange of the therapeutic agent in the external or bulk medium in which the liposomes are suspended with an ammonium ion in internal liposomal compartment. The efficiency of loading depends, at least in part, on an ammonium ion gradient, where the concentration of the ammonium ion inside the liposomes is higher than the concentratration of ammonium ion in the external, bulk suspension medium. The magnitude of this gradient determines to a large extent the level of encapsulation; the larger the gradient, generally the higher the encapsulation.

An ammounium glucuronate gradient across the liposomal lipid bilayer, where the ammonium ion concentration is higher on the inside of the liposomes than in the external suspension medium (i.e., a higher inside/lower outside ammonium ion gradient) may be formed in a variety of ways, *e.g.,* by (i) controlled dilution of the external medium, (ii) dialysis against the desired final medium, (iii) molecular-sieve chromatography, *e.g.,* using Sephadex G-50, against the desired medium, or (iv) high-speed centrifugation and resuspension of pelleted liposomes in the desired final medium. The final external medium selected will depend on the mechanism of gradient formation and the external ion concentration desired. The gradient is measured as the ratio of ammonium glucuronate inside to that outside of the liposomes. Generally, the gradient is in the range of 1000-10 inside/outside. Preferably, the gradient is in the range of 500-50.

The concentration of ammonium glucuronate in an external medium that also contains electrolytes may be measured as ammonia concentration at pH 13-14 (Bolotin, E.M., et al., Journal of Liposome Research 4(I):455-479 (1994)) by an ion analyzer, *e.g.,* a Coming 250 pH/ion analyzer (Coming Science Products, Corning, NY) equipped with a Corning 476130 ammonia electrode and an automatic temperature compensation (ATC) stainless steel probe. If the final external medium lacks electrolytes the ammonium glucuronate gradient may be confirmed by conductivity measurements using a conductivity meter, e.g., a type CDM3 conductivity meter equipped with a CDC 304 immersion electrode with manual temperature compensator type CDA 100 (Radiometer, Copenhagen, Denmark).

In one approach, the ammonium ion gradient is created by controlled dilution. This method gives a diluted liposome preparation. After sizing, the liposomal suspension has a selected first concentration of ammonium glucuronate inside the liposome and in the external bulk medium. The external bulk medium is diluted with a second medium containing no ammonium glucuronate. Exemplary second medium include aqueous solutions containing electrolytes (sodium chloride or potassium chloride) or aqueous solutions containing non electrolytes (glucose or sucrose). The internal and external: media are preferably selected to contain about the same osmolarity, e.g., by suitable adjustment of the concentration of buffer, salt, or low molecular weight solute, such as sucrose. A preferred second medium is 15 mM HEPES buffer containing 5% dextrose at approximately pH 7.

In another approach, a proton gradient across the lipid bilayer is produced by dialysis in which the external bulk medium is exchanged for one lacking ammonium ions, e.g., the same buffer but one in which ammonium glucuronate is replaced by a salt such as NaCl or KCl, or by a sugar that gives the same osmolarity inside and outside of the liposomes. For small-scale preparation, the gradient can be created by four consecutive dialysis exchanges against 25 volumes of the dialysis buffer. For large-scale preparation, the gradient may be prepared by a three-step tangential flow dialysis, e.g., using a Minitan ultrafiltration system (Millipore Corp., Bedford, MA) equipped with "300 K" polysulfone membranes. The dialysis buffer contain electrolytes (e.g., sodium chloride or potassium chloride) or non electrolytes (glucose or sucrose). In one preferred embodiment, the dialysis buffer is 15 mM HEPES containing 5% dextrose at approximately pH 7. Using either of the dialysis approaches (large or small-scale) and under conditions in which the hydration medium was 60-250 mM ammonium glucuronate, a gradient of 1,000 or higher can be obtained without dilution of the liposomal dispersion.

The ionization events that occur when loading an ionizable drug into liposomes against an ammonium ion gradient are described in the art (see U.S. Patent No. 5, 192,549). Briefly, after formation of the liposomes and establishment of a gradient across the liposomal bilayers, ammonium ions inside the liposomes dissociate and are in equilibrium with ammonia and protons. Ammonia gas is permeable in the lipid bilayer, with a permeability coefficient of around 1.3 x 10⁻¹ cm/second, and is able to permeate the liposomal bilayer. The efflux of ammonia shifts the equilibrium within the liposome towad production of protons which results in a [H⁺] gradient, with the intraliposomal concentration higher than that in the extraliposomal medium. Unprotonated drug crosses the liposomal bilayer, becomes protonated inside the liposome, and is stabilized by the anions present in the internal aqueous compartment of the liposome. Formation of a drug-glucuronate salt elevates the intraliposomal pH and induces formation of NH₃ inside the liposmes. This cycle repeats repeated until essentially all the ammonium ions are effluxed from the liposomal internal compartment as NH₃. A therapeutic agent, e.g., doxorubicin, may be loaded into the liposomes by adding a solution of the agent to a suspension of liposomes having an ammonium ion gradient across the liposomal membranes. The suspension is treated under conditions effective to allow passage of the compound from the external medium into the liposomes. Incubation conditions suitable for drug loading are those which (i) allow diffusion of the compound, which is in an uncharged form, into the liposomes, and (ii) preferably lead to high drug loading concentration, e.g., 5-500 mM drug encapsulated, more preferably between 20-300 mM, most preferably between 50-200 mM.

The loading is preferably carried out at a temperature above the phase transition temperature of the liposome lipids. Thus, for liposomes formed predominantly of saturated phospholipids, the loading temperature may be as high as 60°C or more. The loading period is typically between 15-120 minutes, depending on permeability of the drug to the liposome bilayer membrane, temperature, and the relative concentrations of liposome lipid and drug. In one preferred embodiment, the loading is performed at 60°C and for 60 minutes.

Thus, with proper selection of liposome concentration, external concentration of added compound, and the ion gradient, essentially all of the added compound may be loaded into the liposomes. For example, with an ammonium ion gradient of approximately 1000, encapsulation of doxorubicin can be greater than 90%. Knowing the calculated internal liposome volume, and the maximum concentration of loaded drug, one can then select an amount of drug in the external medium which leads to substantially complete loading into the liposomes.

If drug loading is not effective to substantially deplete the external medium of free drug, the liposome suspension may be treated, following drug loading, to remove non-encapsulated drug. Free drug can be removed, for example, by ion exchange chromatography, molecular sieve chromatography, dialysis, or centrifugation. In one embodiment, the non-entrapped drug is removed using Dowex 50WX-4 (Dow Chemical, MI). For example, free doxorubicin (but not liposomal doxorubicin) binds to a cation exchange resin (Storm, G. et al., Biochim Biophys Acta, 818:343 (1985)).

### II. In vitro Characterization

### A. In vitro Cytotoxicily

The *in vitro* cytotoxicity of free doxorubicin (free-DOX), of liposome-entrapped doxorubicin loaded against an ammonium sulfate gradient (lipo-dox-AS) or against an ammonium glucuronate gradient (lipo-dox-AG) were tested against two mouse cell lines (M109-S and M109-R) and three human tumor cell lines (C-26, KB, and KB-V). M109-R and KB-V cell lines are doxorubicin-resistant sublines of M109-S and KB, respectively. The cells were exposed continuously to the drug formulation for 72 hours following the experimental details described by Horowitz, et al., Biochimica et Biophysica Acta, 1109:203-209 (1992) and also in Example 2.

Table 1 shows the doxorubicin concentration needed to inhibit 50% of cell grow (IC50 values) for free doxorubicin (F-DOX), lipo-dox-AG, and lipo-dox-AS. Doxorubicin in free from is more cytotoxic than either of the two liposomal doxorubicin formulations. Doxorubicin loaded into liposomes against an ammonium glucuronate gradient is more cytotoxic than when loaded into liposomes against an ammonium sulfate gradient, suggesting that the drug is more bioavailable from a glucuronate salt than from a sulfate salt.

**Table 1: Inhibitory Concentration (IC50) Values**

| | IC50 (µM) | | |
|---|---|---|---|
| Cell Line | F-DOX | Lipo-DOX-AS | Lipo-DOX-AG |
| M109-S | 0.56 | 9.8 | 1.4 |
| M109-R | 2.00 | >300 | 28.0 |
| KB | 0.04 | 7.6 | 1.4 |
| KB-V | 0.69 | >300 | 21.0 |
| C26 | 0.96 | >200 | 64.0 |

That lipo-dox-AG is more cytotoxic than lipo-dox-AS is further demonstrated by the inhibition curves shown in Figs. 1A-1E, which show the growth rate of the cells, as a percent of cells not treated with drug (control), against the amount of doxorubicin added to the growth medium. Figs. 1A-1E are inhibition curves for the mouse cell lines, M109-S (Fig. 1A), M109-R (Fig. 1B) and the human cell lines C-26 (Fig. 1C), KB (Fig. 1D), and KB-V (Fig. 1E). The doxorubicin concentration, in nM, of the different formulations are represented as free doxorubicin (circles), lipo-dox-AG (squares), and lipo-dox-AS (triangles). All the drug formulations at doxorubicin concentrations between 10² to 10⁶ were cytotoxic to each of the tumor cell lines tested. In all cases, with variations in the growth rate inhibition, lipo-dox-AG was more cytotoxic than lipo-dox-AS, showing that drug from the liposomal-ammonium glucuronate platform was more readily bioavailable than drug from the liposomal-ammonium sulfate platform.

### B. In vitro Drug Uptake by Tumor Cells

*In vitro* accumulation of doxorubicin in mouse tumor cells was studied by exposing KB, KB-V, and M109-R cells to free doxorubicin, lipo-dox-AS, or lipo-dox-AG for 1, 5, and 24 hours, as described in Example 2. Table 2 shows the results of the study. There is a greater drug accumulation in cells treated with lipo-dox-AG than in those treated with lipo-dox-AS. This is consistent with the *in vitro* cytotoxicity results described above (Figs. 1A-1E), which showed that lipo-dox-AG was more cytotoxic than lipo-dox-AS.

**Table 2: In vitro Uptake of Doxorubicin into Tumor Cells**

| | Doxorubicin Uptake | | |
|---|---|---|---|
| | (ng DOX/10⁶ cells) | | |
| Cell Line, Exposure Time | Free dox | Lipo-dox-AS | Lipo-dox-AG |
| KB, 1 hr | 426 (33) | 5.0 (0.4) | 7.8 (0.6) |
| KB, 5 hr | 937 (46) | 8.8 (0.4) | 15.0 (0.4) |
| KB, 24 hr | 840 (15) | 24.0 (1) | 154.0 (9) |
| KB-V, 1 hr | 311 (22) | 5.4 (0.8) | 9.6 (1.5) |
| KB-V, 5 hr | 931 (21) | 12.3 (1.2) | 18.0 (0.8) |
| M109-R, 24 | 80 (3) | 7.0 (2) | 25.0 (5) |

### C. In vitro Leakage in Plasma

To determine the *in vitro* leakage of liposome-encapsulated drug in plasma, lipo-dox-AS and lipo-dox-AG were incubated in 90% human plasma at 37°C with continuous shaking in incubation flask containing Dowex cation-exchange resin beads. The resin beads bind released drug, whether free or protein bound. At pre-scheduled intervals, samples were taken for acidified alcohol extraction and fluorometric determination of the fraction of drug remaining associated with liposomes (*i*.*e*., not trapped by the resin beads). The results are shown in Fig. 2 and indicate that doxorubicin from lipo-dox-AG from the liposome faster than drug from lipo-dox-AS. The difference between the two preparations begins to manifest after 24 hr of incubation. At end of incubation (96 hr), lipo-dox-AG has released about twice as much doxorubicin as lipo-dox-AS (∼80% vs. 40%).

### III. In Vivo Characterization

### A. Plasma Clearance

The pharmacokinetics of doxorubicin entrapped in liposomes by loading against an ammonium glucoronate gradient were evaluated in 3-month-old BALB/c female mice. As described in Example 3A, liposomes with entrapped doxorubicin loaded against ammonium sulfate or ammonium glucuronate were injected intravenously into the mice. Blood samples were taken at selected intervals and analyzed for doxorubicin concentration. Fig. 3 shows the plasma doxorubicin concentration for mice treated with lipo-dox-AG (cross hatched bars) or with lipo-dox-AS (dotted bars). The half-life of doxorubicin when administered from a lipo-dox-AG platform is approximately 16 hours, while that of doxorubicin when administered from a lipo-dox-AS platform is approximately 24 hours. It is also apparent that lipo-dox-AG is cleared faster than lipo-dox-AS. The lipo-dox-AG blooc concentrations were 25% lower at 4 hours post intravenous administration, 33% lower at 24 hours, and almost 50% lower at 48 hours post intravenous administration. Since the composition and size of the liposomes were identical, the rate of uptake by the reticuloendothelial system (RES.) should be similar. Accordingly, the faster clearance is probably the result of a faster release rate *in vivo* of doxorubicin from the lipo-dox-AG formulation, consistent with the the *in vitro* experiments.

### B. In Vivo Therapeutic Activity

To determine whether the faster clearance of doxorubicin when admininstered from liposomes containing a doxorubicin-glucuronate salt has an impact on therapeutic efficacy, the liposomal formulations were administered to tumor-bearing mice.

As described in Example 3B, mice were inoculated with M109S tumor cells (10⁶ cells) and treated with a single dose of doxorubicin at 10 mg/kg of either free doxorubicin, lipo-dox-AS, or lipo-dox-AG post tumor inoculation. Fig. 4 shows the mean (n=10) footpad thickness, in mm, against days post-doxorubicin treatment. Both liposomal preparations were more effective in suppressing tumor growth than the free drug (circles). There was a slight, but insignificant, improvement in efficacy when mice were treated with lipo-dox-AG (squares) compared to lipo-dox-AS (triangles).

In another study, also described in Example 3B, mice were inoculated with M109R cells (10⁶ cells). Ten days after inoculation, the mice were treated with either free doxorubicin, lipo-dox-AS, or lipo-dox-AG at a dose of 8 mg/kg. The same dose was administered again one week and three weeks later. Fig. 5 shows the mean (n=10) footpad thickness, in mm, as a function of days post tumor inoculation. Both liposomal preparations (triangles, open squares) were more effective in inhibiting tumor growth than free drug (circles), despite the progressive tumor growth in all test groups, probably due to the resistant nature of this tumor.

In another study, also described in Example 3B, mice were inoculated with C-26 cells (10⁶ cells) to induce a tumor and treated, five days after tumor inoculation, with either free doxorubicin, lipo-dox-AS, or lipo-dox-AG at a doxorubicin dose of 10 mg/kg. Fig. 6 shows the number of surviving mice as a function of time post tumor inoculation. Untreated (control) mice died quickly with a median survival of 13 days (not shown). Mice treated with free doxorubicin (circles) showed a neglible increase in mean survival time (4 days more than control, *i*.*e*., 17 days). Both liposomal preparations (squares, triangles) were more effective in extending survival time in tumor-bearing mice than was free doxorubicin.

All the above models are carcinoma-type tumors. An additional model tested (results not shown) was the J6456 lymphoma of BALB/c mice with an experimental design similar to the C-26 model (intraperitoneal 10⁶ tumor cells, intravenous therapy with a dose of 10 mg/kg on day 5 post-tumor inoculation). The liposomal formulations were more effective than free drug, with no significant differences between mouse survival time after treatment with lipo-dox-AS or lipo-dox-AG.

From the foregoing, it can be seen how various objects and features of the invention are met. Liposomes having a drug entrapped in the form of a glucuronate salt provide a higher release rate of drug than does a similar liposome where the drug is entrapped in the form of a sulfate salt, without significant effect on drug efficacy. Clinical data with liposome-entrapped doxorubicin (Doxil^{®}) indicate that the incidence and severity of PPE decrease with a shortening of the circulation half-life of Doxil^{®}, the faster release, and shorter circulation of doxorubicin in the form of lipo-dox-AG provides a good alternative for doxorubicin delivery. It will be appreciated that the findings specific to doxorubicin extend to other drugs capable of remote loading against an ammonium ion gradient, such as those recited herein.

### IV. Examples

The following examples further illustrate the invention described herein and are in no way intended to limit the scope of the invention.

### EXAMPLE 1

### Liposome Preparation and Loading

### A. Liposome Preparation

Liposomes containing ammonium glucuronate in the aqueous compartments were prepared as follows. The lipid component, hydrogenated soy phosphatidylcholine (HSPC), cholesterol and methoxy-capped polyethylene glycol derivatized distearyl phosphatidylethanolamine (mPEG(200)-DSPE) in a molar ratio of 92.5:70:7.5, were dissolved in chloroform. The solvent was evaporated using a rotary evaporator under reduced pressure leaving behind a dried lipid thin film. The dried lipid thin film was hydrated with a 250 mM aqueous ammonium glucuronate buffer solution (pH 5.5), forming liposomes containing ammonium glucuronate in the internal aqueous compartments and suspended in an ammonium glucuronate external bulk medium. The liposomes were then sized by extrusion through 0.5 µm pore size membranes.

Following extrusion, the external ammonium glucuronate buffer was exchanged by dialysis against a dialysis buffer containing 5% dextrose and 15 mM Hepes at pH 7.

A comparative liposome formulation containing 250 mM ammonium sulfate in the interior aqueous compartments was similarly prepared by using 250 µm ammonium sulfate as the hydration buffer. The batches obtained were similar to the ammonium glucuronate preparations in vesicle size, drug-loading efficiency, and drug-to-phospholipid ratio.

### B. Remote Loading

Doxorubicin was loaded into the liposomes containing ammonium glucuronate (lipo-dox-AG) and into the liposomes containing ammonium sulfate (lipo-dox-AS) by incubating the liposomes prepared as described in A. above with a solution of doxorubicin for 1 hour at 60°C. Encapsulation of doxorubicin proceded to >90% efficency. The final drug to phospholipid ratio was 100-150 µg/µmol.

Free doxorubicin (i.e., doxorubicin not entrapped in a liposome) in the external bulk medium was removed by chromatography on a Sephadex G-50 column eluted with degassed dextrose-Hepes buffer.

### EXAMPLE 2

### In vitro Characterization

### A. In vitro Cytotoxicity

Free doxorubicin and liposomal formulations of doxorubicin, prepared as described above in Example 1, were tested against five mouse and human tumor cell lines (M109-S, M109-R, C-26, KB, KB-V).

Cells for each line were exposed continuously to drug for 72 hours. Other experimental details were as described by Horowitz et a/., Biochem Biophys Acta, 1109(2):203 (1992). Briefly, 5x10³ cells from exponentially growing cultures in 200 µL aliquots were plated onto 96-well flat-bottom microtiter plates. Following 20 hours in culture, during which cells attached and resumed growth, 20 µL of the tested drug formulation (free doxorubicin, lipo-dox-AS, lipo-dox-AG) were added to each well. For each 10-fold increase in drug concentration, six drug concentration points were tested. Each test was performed in triplicate wells and in two parallel plates. The cells were treated continously for 72 hours. The cultures were fixed by the addition of 50 µL 2.5% glutaraldehyde to each well for 10 minutes. The plates were washed three times with de-ionized water, once with 0.1 M borate buffer (pH 8.5) and then stained for 60 minutes with 100 µL methylene blue (1% in 0.1 M buffer borate, pH 8.5) at room temperature. The plates were rinsed in five baths of de-ionized water to remove non-cell bound dye and were then dried. The dye was extracted with 200 µL 0.1 M HCl for 60 min at 37°C and the optical density was determined using a microplate spectrophotometer.

The growth rate was calculated by dividing the doubling times of drug-treated cells with those of the control cells. The drug concentration which caused a 50% inhibition of the control growth rate (IC50) was calculated yb interpolation of the two closest values of the growth inhibition curve.

Table 1 shows the IC50 values for free doxorubicin, lipo-dox-AS, and lipo-dox-AG for each of the cell lines, and the corresponding growth inhibition curves are shown in Figs. 1A-1E.

### B. In vitro Drug Updake

Cellular accumulation of doxorubicin was assayed by a method similar to that described in Chambers, S. K et al., Cancer Res., 49:6275-6279 (1989). Monolayers of KB, KB-V, and M109-R cells (exponentially growing cultures of about 10⁶ cells in 35-mm plates) were incubated with free doxorubicin, lipo-dox-AS, and lipo-dox-AG for 1, 5, and 24 hours. At the end of the incubation, the cells were rinsed three times with PBS and the drug was extracted from the cells with 1 mL acidified isopropanol (0.075 M HC1 in 90% isopropanol), for 20 hours at 4°C. Doxorubicin concentration was determined spectrofluorometrically using an excitation wavelength of 470 nm and an emission wavelength of 590 nm. The fluorescence intensity emitted was translated into doxorubicin-equivalents based on a doxorubicin standard curve, after readings of untreated background cells were subtracted.

The result of drug uptake by KB, KB-V, and M109-R cells after exposure to free doxorubicin, lipo-dox-AS, and lipo-dox-AG for 1, 5, and 24 hr are shown in Table 2.

### C. In vitro Plasma Leakage.

### Materials

Lipo-dox-AS and lipo-dox-AG were prepared as described in Example 1 at a concentration of >500 µg doxorubicin/mL.

2 mL of 50% Dowex^{®} cation exchage resin beads (Sigma, 50W-hydrogen, 50% pre-cleaned in saline) were added to 15 mL plastic tissue culture round bottom tubes. The tubes were centrifuged for 10 min at 2,000 rpm (850g), and the liquid was decanted. The liposomal preparations were diluted with human plasma to approximately 5 µg doxorubicin/mL in 90% human plasma. Duplicate tubes for each liposomal preparation were prepared, and tubes containing the liposomal preparations absent Dowex resin beads were prepared.

An acidic isopropanol solution was prepared from 10% 0.75N HCl in 90% isopropanol, volume/volume. The reagents were reagent grade chemicals obtainable from Sigma.

All the materials used in the study were sterile, and all the experiments were performed in sterile conditions.

### Assay

Dowex® cation exchange resin beads bind doxorubicin in human plasma whether the drug is free or protein bound. In this assay lipo-dox-AG and lipo-dox-AS were incubated in the tubes containing the resid beads and human plasma (as described above) at 37°C with continuous shaking using a rotary shaker to prevent sedimentation of the resin beads. At prescheduled intervals, samples were taken for acidified alcohol extraction and fluorometric determination of the fraction of drug remaining associated with liposomes (*i*.*e*., not trapped by the resin beads). The following stepwise protocol for the analysis was followed.
1. Add 30 mL of human plasma into a 50 mL-tube.
2. Add 2 mL of 50% sterile Dowex® resin beads in saline to the centrifugation tubes (15 mL, plastic round bottom) and centrifuge for 10 min 2,000 rpm (850g). Decant the supernatant fluid from the centrifuge tubes.
3. Using liposomal preparations prepared as described in Example 1, add an amount of the liposome suspension to the 50 mL tubes containing 30 mL human plasma to obtain a stock solution having a final concentration of 5 µg/mL doxorubicin.
4. Add 9 mL of the 5 µg/mL doxorubicin liposomal suspension stock solution to each of the centrifuge tubes containing resin beads (Tube Nos. A, B), and add 10 mL of the liposomal stock solution to an tube absent any resin beads (Tube no. C). Mix.
5. Remove 1 mL aliquots from each tube (A, B, C) and centrifuge for 3 minutes at 14,000 rpm. Remove 200 µL from the supernatants for a time zero reading, and freeze the samples at -20°C until analysis.
6. Incubate the tubes at 37°C with continuous shaking on a rotary shaker that grips and rotates the tubes 360°C at slow motion, with sufficient speed to prevent sedimentation of the resin beads.
7. Remove a 1 mL aliquot from each of the tubes at 1, 4, 24, 48, 72, and 96 hours. Centrifuge each aliquot at 14,000 rpm for 3 minutes, remove a 200 µL aliquot of the clear supernatant. Freeze the aliquot at about -20°C until analysis.
8. For analysis of the samples, 1.8 mL of acidified isopropanol was added to to the 200 µL samples to extract doxorubicin from the liposomes. The samples were incubated overnight at 4°C, and then centrifuged to remove the precipitate (2,000 rpm for 10 minutes). The clear supernatants were examined in a spectrofluorimeter equipped with high wavelength photomultiplier, excitation at 470 nm and emission at 590 nm. Doxorubicin concentration was determined based on a standard calibration curve, where the concentration obtained represented the amount of doxorubicin retained in the liposomes.

The results are shown in Fig. 2.

### EXAMPLE 3

### In vivo Characterization

### A. In vivo Plasma Clearance Rate

Three month-old BALB/c female mice were injected intravenously with 10 mg/kg of either lipo-dox-AS or with lipo-dox-AG, prepared as described in Example 1. Blood samples were taken 4, 24 and 48 hours after injection for analysis of plasma doxorubicin levels. The results are shown in Fig. 3.

### B. In Vivo Therapeutic Activity.

Thirty mice were inoculated in the footpad with M109-S cells (10⁶ cells). Seven days later, when the footpad thickness increased from a normal value of approximately 1.5 mm to an average of 2.0-2.5 mm, the mice were divided into three groups of 10 each and the mice groups were injected intravenously with either free doxorubicin, lipo-dox-AS, or lipo-dox-AG at a doxorubicin dose of 10 mg/kg. Thereafter, the footpad thickness was measured twice a week with alipers to follow tumor growth and effect of therapy. The results are shown in Fig. 4.

In a separate study, thirty mice were inoculated in the footpad with the doxorubicin-resistant tumor cell line M109R cells (10⁶ cells). Ten days later, when the footpad thickness increased from a normal value of approximately 1.5 mm to an average of 2.0-2.5 mm, the mice were divided into three groups for intravenous treatment with free doxorubicin, lipo-dox-AS, or lipo-dox-AG at a doxorubicin dose of 8 mg/kg. Two additional injections were given at the same dose 1 week and 3 weeks later. The footpad thickness was measured twice a week with calipers and the results are shown in Fig. 5.

In another study, mice were inoculated i.p. with C-26 cells (10⁶ cells). Five days later, the mice were separated into three groups of 10 mice each, and each group of mice was injected intravenously with either free doxorubicin, lipo-dox-AS, or lipo-dox-AG at a dose of 10 mg/kg. The survival of these mice was followed and survival curves are shown in Fig. 6.

Although the invention has been described with respect to particular embodiments, it will be apparent to those skilled in the art that various changes and modifications can be made without departing from the invention.

## Claims

1. A liposome composition, comprising
liposomes comprised of vesicle forming lipids and having an entrapped ionizable therapeutic agent in association with a glucuronate anion.

2. The composition of claim 1, wherein said vesicle-forming lipids are phospholipids.

3. The composition of claim 1, wherein said liposomes further comprise between about 1-20 mole percent of a vesicle-forming lipid derivatized with a hydrophilic polymer.

4. The composition of claim 3, wherein said hydrophilic polymer is polyethylene glycol.

5. The composition of claim 3, wherein said vesicle-forming lipid is hydrogenated soy phosphatidylcholine (HSPC) and said vesicle-forming lipid derivatized with a hydrophilic polymer is distearoyl phosphatidylethanolamine (DSPE) derivatized with polyethylene glycol.

6. The composition of claim 5, wherein said liposomes further comprise cholesterol.

7. The composition of claim 5, wherein said liposomes are comprised of HSPC, cholesterol, and DSPE-PEG in a molar ratio of is 92.5:70:7.5.

8. The composition of claim 1, claim 3, or claim 5, wherein said therapeutic agent is an anthracycline antibiotic.

9. The composition of claim 8, wherein said antibiotic is selected from doxorubicin, daunorubicin, and epirubicin.

10. The composition according to any one of claims 1-9 for use in treating a patient.

11. The composition according to any one of claims 1-9 for use in treating a neoplasm in a patient.

12. A method of preparing liposomes having an entrapped ionizable therapeutic agent, where said therapeutic agent is loaded into pre-formed liposomes against an ammonium ion gradient with sulfate as a counterion, the improvement comprising
loading the ionizable therapeutic agent into liposomes by an ammonium ion gradient having glucuronate as a counterion.

13. The method of claim 12, wherein said loading includes preparing a suspension of liposomes, each liposome having at least one internal aqueous compartment that contains ammonium glucuronate at a first concentration.

14. The method of claim 13, wherein said preparing a suspension of liposomes includes preparing liposomes suspended in an external bulk medium having a second concentration of ammonium glucuronate, wherein the first concentration is higher than the second concentration thereby establishing an ammonium ion concentration gradient across lipid bilayers of the liposomes.

15. The method of claim 14, further comprising adding an amount of the therapeutic agent to the suspension of liposomes.

16. The method of claim 15, wherein said adding comprises adding an anthracycline antibiotic.

17. A method of preparing liposomes, comprising
forming liposomes having an internal compartment and a bilayer lipid membrane, said liposomes having a concentration gradient of ammonium glucuronate across their bilayer lipid membranes;
contacting the liposomes with an ionizable therapeutic agent to achieve transport of the agent into the internal compartment.

18. The method of claim 17, wherein said contacting comprises contacting the liposomes with an ionizable anthracycline therapeutic agent.

19. The method of claim 18, wherein said contacting comprises contacting the liposomes with an ionizable anthracycline therapeutic agent selected from doxorubicin, daunorubicin, and epirubicin.

20. The method of claim 17, wherein said forming liposomes includes (i) preparing a suspension of liposomes, each liposome in the suspension having at least one internal aqueous compartment that contains ammonium glucuronate at a first concentration, said liposomes suspended in an external bulk medium comprising ammonium glucuronate at the first concentration; (ii) reducing the first concentration of ammonium glucuronate in the external bulk medium to a lower, second concentration of ammonium glucuronate, thereby establishing an ammonium ion concentration gradient across lipid bilayers of the liposomes.

21. The method of claim 20, wherein said reducing is achieved by dilution, dialysis, diafiltration, or ion exchange.

22. A method for loading a protonatable compound into pre-formed liposomes, comprising:
preparing a suspension of liposomes having a greater concentration of ammonium glucuronate inside the liposomes than outside the liposomes thereby establishing an ammonium ion concentration gradient from the inside to outside of the liposomes; wherein said gradient is capable of active transport of said protonatable compound towards the inside of the liposomes,
adding an amount of protonatable compound to the suspension, and
allowing said protonatable compound to transport into said liposomes to achieve a content of said protonatable compound inside the liposomes to be greater than that outside of the liposomes.

23. The method of claim 22, wherein said preparing comprises
forming the liposomes in the presence of an ammonium glucuronate solution having a first concentration;
entrapping said ammonium glucuronate solution of said first concentration inside said liposomes; and
reducing said first concentration of said ammonium glucuronate solution outside of the liposomes to a second concentration which is less than that of said first concentration.

24. The method of claim 23, wherein said protonatable compound is an anthracycline antibiotic.

25. The method of claim 24, wherein said anthracycline antibiotic is doxorubicin or daunorubicin.

## Patentansprüche

1. Liposomzusammensetzung umfassend
Liposomen, die Vesikel-bildende Lipide aufweisen und ein eingeschlossenes ionisierbares therapeutisches Mittel in Verbindung mit einem Glucuronatanion haben.

2. Verbindung nach Anspruch 1, in der die Vesikel-bildenden Lipide Phospholipide sind.

3. Verbindung nach Anspruch 1, in der die Liposomen des weiteren zwischen etwa 1 bis 20 Molprozent eines Vesikel-bildendendes Lipids umfassen, das mit einem hydrophilen Polymer derivatisiert ist.

4. Verbindung nach Anspruch 3, bei der das hydrophile Polymer Polyethylenglykol ist.

5. Verbindung nach Anspruch 3, in der das Vesikel-bildende Lipid hydriertes Soja-Phosphatidylcholin (HSPC) ist und das mit einem hydrophilen Polymer derivatisierte Vesikel-bildende Lipid ist mit Polyethylenglykol derivatisiertes Distearoylphosphatidylethanolamin (DSPE).

6. Verbindung nach Anspruch 5, in der die Liposomen desweiteren Cholesterin umfassen.

7. Verbindung nach Anspruch 6, in der die Liposomen HSPC, Cholesterin und DSPE-PEG in einem molaren Verhältnis von 92,5 : 70 : 7,5 umfassen

8. Verbindung nach Anspruch 1, Anspruch 3 oder Anspruch 5, in der das therapeutische Mittel ein Anthracyclinantibiotikum ist.

9. Verbindung nach Anspruch 8, in der das Antibiotikum ausgewählt wird aus Doxorubicin, Daunorubicin und Epirubicin.

10. Verbindung nach einem der Ansprüche 1 bis 9 zur Behandlung eines Patienten.

11. Verbindung nach einem der Ansprüche 1 bis 9 zur Behandlung eines Neoplasma bei einem Patienten.

12. Verfahren zur Herstellung von Liposomen mit einem eingeschlossenen therapeutischen Mittel, wobei vorgeformte Liposomen gegen einen Ammoniumionengradienten mit Sulfat als Gegenion mit dem therapeutischen Mittel beladen werden, die Verbesserung umfaßt
Beladung des ionisierbaren therapeutischen Mittels in Liposomen mit einem Ammoniumionengradienten mit Glucuronat als ein Gegenion.

13. Verfahren nach Anspruch 12, wobei die Beladung die Herstellung einer Suspension aus Liposomen umfaßt, jedes Liposom hat wenigstens ein inneres wässeriges Kompartiment, das Ammoniumglucuronat mit einer ersten Konzentration enthält.

14. Verfahren nach Anspruch 13, wobei die Herstellung einer Suspension aus Liposomen die Herstellung von Liposomen einschließt, die in einem externen Grundmedium mit einer zweiten Konzentration Ammoniumglucuronat suspendiert sind, wobei die erste Konzentration höher ist als die zweite Konzentration, wodurch ein Ammonioumionengradient über die Lipiddoppelschicht der Lipososmen geschaffen wird.

15. Verfahren nach Anspruch 14, das des weiteren die Zugabe einer Menge des therapeutischen Mittels zu der Liposomensuspension umfaßt.

16. Verfahren nach Anspruch 15, bei dem die Zugabe die Zugabe eines Anthracyclinantibiotikums umfaßt.

17. Verfahren zur Herstellung von Liposomen umfassend
Bildung von Liposomen mit einem inneren Kompartiment und einerDoppelschichtlipidmembran, die Liposomen haben einen Konzentrationsgradienten mit Ammoniumglucuronat über ihre Doppelschichtlipidmembranen;
Inkontaktbringen der Liposomen mit einem ionisierbaren therapeutischen Mittel, um den Transport des Mittels in das innere Kompartiment zu erreichen.

18. Verfahren nach Anspruch 17, wobei das Inkontaktbringen umfaßt, daß die Liposomen mit einem ionisierbaren Anthracyclin-therapeutischen Mittel in Kontakt gebracht werden.

19. Verfahren nach Anspruch 18, wobei wobei das Inkontaktbringen umfaßt, daß die Liposomen mit einem ionisierbaren Anthracyclin-therapeutischen Mittel ausgewählt aus Doxorubicin, Daunorobicin und Epirubicin in Kontakt gebracht werden.

20. Verfahren nach Anspruch 17, wobei die Bildung der Liposomen einschließt (i) Herstellung einer Suspension mit Liposomen, jedes Liposom in der Suspension hat wenigstens ein inneres wässeriges Kompartiment, das Ammoniumglucuronat mit einer ersten Konzentration enthält, die Liposomen sind in einem externen Grundmedium suspendiert, das Ammoniumglucuronat mit einer ersten Konzentration umfaßt; (ii) Reduzierung der ersten Konzentration an Ammoniumglucuronat in dem externen Grundmedium auf eine niedrigere zweite Konzentration an Ammoniumglucuronat, wodurch ein Konzentrationsgradient von Ammoniumionen über die Lipiddoppelschichten der Liposomen geschaffen wird.

21. Verfahren nach Anspruch 20, wobei die Reduzierung durch Verdünnung, Dialyse, Diafiltration oder Ionenaustausch erreicht wird.

22. Verfahren zur Zuführung einer protonierbaren Verbindung in vorgeformte Liposomen umfassend:
Herstellung einer Suspension mit Liposomen mit einer größeren Konzentration an Ammoniumglucuronat innerhalb als außerhalb der Liposomen, wodurch ein Konzentrationsgradient von Ammoniumionen vom Innern zum Äußern der Liposomen geschaffen wird, wobei der Gradient zum aktiven Transport der protonierbaren Verbindung in Richtung auf das Innere der Liposomen in der Lage ist;
Zugabe einer Menge der protonierbaren Verbindung zu der Suspension, und
Ermöglichen, daß die protonierbare Verbindung in die Liposomen transportiert wird, um einen Gehalt der protonierbaren Verbindung innerhalb der Liposomen zu erreichen, der größer als der außerhalb der Liposomen ist.

23. Verfahren nach Anspruch 22, wobei die Herstellung umfaßt
Bildung der Liposomen in Gegenwart einer Ammoniumglucuronatlösung mit einer ersten Konzentration;
Einschließen der Ammoniumglucuronatlösung mit der ersten Konzentration im Innern der Liposomen; und
Reduzieren der ersten Konzentration der Ammoniumglucuronatlösung außerhalb der Liposomen auf eine zweite Konzentration, die geringer ist als die erste Konzentration.

24. Verfahren nach Anspruch 23, wobei die protonierbare Verbindung ein Anthracyclinantibiotikum ist.

25. Verfahren nach Anspruch 24, wobei das Anthracyclinantibiotikum Doxorubicin oder Daunorobicin ist.

## Revendications

1. - Composition de liposomes, comprenant des liposomes composés de lipides formant des vésicules et ayant un agent thérapeutique ionisable piégé en association avec un anion glucuronate.

2. - Composition selon la revendication 1, dans laquelle lesdits lipides formant des vésicules sont des phospholipides.

3. - Composition selon la revendication 1, dans laquelle lesdits liposomes comprennent en outre entre environ 1-20 pour cent en moles d'un lipide formant des vésicules transformé en dérivé par un polymère hydrophile.

4. - Composition selon la revendication 3, dans laquelle ledit polymère hydrophile est le polyéthylène glycol.

5. - Composition selon la revendication 3, dans laquelle ledit lipide formant des vésicules est la phosphatidylcholine de soja hydrogénée (HSPC) et ledit lipide formant des vésicules, transformé en dérivé par un polymère hydrophile, est la distéaroyl phosphatidyléthanolamine (DSPE) transformée en dérivé par le polyéthylène glycol.

6. - Composition selon la revendication 5, dans laquelle lesdits liposomes comprennent en outre le cholestérol.

7. - Composition selon la revendication 5, dans laquelle lesdits liposomes sont composés de HSPC, de cholestérol et de DSPE-PEG dans un rapport molaire de 92,5:70:7,5.

8. - Composition selon la revendication 1, la revendication 3 ou la revendication 5, dans laquelle ledit agent thérapeutique est un antibiotique anthracycline.

9. - Composition selon la revendication 8, dans laquelle ledit antibiotique est choisi parmi la doxorubicine, la daunorubicine et l'épirubicine.

10. - Composition selon l'une quelconque des revendications 1-9, destinée à être utilisée dans le traitement d'un patient.

11. - Composition selon l'une quelconque des revendications 1-9, destinée à être utilisée dans le traitement d'un néoplasme dans un patient.

12. - Procédé de préparation de liposomes ayant un agent thérapeutique ionisable piégé, ledit agent thérapeutique étant chargé dans des liposomes préformés contre un gradient d'ions ammonium avec le sulfate comme contre-ion, le perfectionnement comprenant
l'opération consistant à charger l'agent thérapeutique ionisable dans des liposomes par un gradient d'ions ammonium ayant le glucuronate comme contre-ion.

13. - Procédé selon la revendication 12, dans lequel ladite opération comprend la préparation d'une suspension de liposomes, chaque liposome ayant au moins un compartiment aqueux interne qui contient du glucuronate d'ammonium à une première concentration.

14. - Procédé selon la revendication 13, dans lequel ladite préparation d'une suspension de liposomes comprend la préparation de liposomes en suspension dans un milieu en vrac externe ayant une seconde concentration de glucuronate d'ammonium, la première concentration étant supérieure à la seconde concentration, permettant ainsi d'établir un gradient de concentration d'ions ammonium à travers les bicouches lipidiques des liposomes.

15. - Procédé selon la revendication 14, comprenant en outre l'addition d'une quantité de l'agent thérapeutique à la suspension de liposomes.

16. - Procédé selon la revendication 15, dans lequel ladite addition comprend l'addition d'un antibiotique anthracycline.

17. - Procédé de préparation de liposomes, comprenant les opérations consistant à :
- former des liposomes ayant un compartiment interne et une membrane lipidique bicouche, lesdits liposomes ayant un gradient de concentration de glucuronate d'ammonium à travers leurs membranes lipidiques bicouches ;
- mettre en contact les liposomes avec un agent thérapeutique ionisable pour réaliser le transport de l'agent dans le compartiment interne.

18. - Procédé selon la revendication 17, dans lequel ladite mise en contact comprend la mise en contact des liposomes avec un agent thérapeutique anthracycline ionisable.

19. - Procédé selon la revendication 18, dans lequel ladite mise en contact comprend la mise en contact des liposomes avec un agent thérapeutique anthracycline ionisable choisi parmi la doxorubicine, la daunorubicine et l'épirubicine.

20. - Procédé selon la revendication 17, dans lequel ladite formation de liposomes comprend les opérations consistant à (i) préparer une suspension de liposomes, chaque liposome dans la suspension ayant au moins un compartiment aqueux interne qui contient du glucuronate d'ammonium à une première concentration, lesdits liposomes en suspension dans un milieu en vrac externe comprenant du glucuronate d'ammonium à la première concentration ; (ii) réduire la première concentration de glucuronate d'ammonium dans le milieu en vrac externe à une seconde concentration, inférieure, de glucuronate d'ammonium, permettant ainsi d'établir un gradient de concentration d'ions ammonium à travers les bicouches lipidiques des liposomes.

21. - Procédé selon la revendication 20, dans lequel ladite réduction est obtenue par dilution, dialyse, diafiltration ou échange d'ions.

22. - Procédé pour charger un composé protonable dans des liposomes préformés, comprenant les opérations consistant à :
- préparer une suspension de liposomes ayant une concentration de glucuronate d'ammonium plus grande à l'intérieur des liposomes qu'à l'extérieur des liposomes, permettant ainsi d'établir un gradient de concentration d'ions ammonium de l'intérieur à l'extérieur des liposomes ; ledit gradient étant apte à permettre un transport actif dudit composé protonable vers l'intérieur des liposomes ;
- ajouter une quantité de composé protonable à la suspension ; et
- amener ledit composé protonable à se transporter à l'intérieur desdits liposomes pour parvenir à ce qu'une teneur dudit composé protonable à l'intérieur des liposomes soit supérieure à celle à l'extérieur des liposomes.

23. - Procédé selon la revendication 22, dans lequel ladite préparation comprend les opérations consistant à :
- former les liposomes en présence d'une solution de glucuronate d'ammonium ayant une première concentration ;
- piéger ladite solution de glucuronate d'ammonium de ladite première concentration à l'intérieur desdits liposomes ; et
- réduire ladite première concentration de ladite solution de glucuronate d'ammonium à l'extérieur des liposomes à une seconde concentration qui est inférieure à celle de ladite première concentration.

24. - Procédé selon la revendication 23, dans lequel ledit composé protonable est un antibiotique anthracycline.

25. - Procédé selon la revendication 24, dans lequel ledit antibiotique anthracycline est la doxorubicine ou la daunorubicine.
